# EUROPÄISCHE PATENTSCHRIFT

(11) **EP 1 024 131 B1**
(45) Veröffentlichungstag und Bekanntmachung des Hinweises auf die Patenterteilung: **25.09.2002**
(21) Anmeldenummer: 00100474.6
(22) Anmeldetag: 11.01.2000
(51) Int. Cl.: C07C 67/00, C07C 69/017

(54) **Verfahren zur Herstellung von 2,3,5-Trimethylhydrochinondiestern**
Process for the preparation of 2,3,5-trimethylhydroquinonediesters
Procédé de préparation de diesters de la 2,3,5-triméthylhydrochinone

(30) Priorität: 28.01.1999 DE 19903269
(43) Veröffentlichungstag der Anmeldung: 02.08.2000
(73) Patentinhaber: Degussa AG, 40474 Düsseldorf (DE)
(72) Erfinder: Krill, Steffen, Dr., 67346 Speyer (DE); Weigel, Horst, 63517 Rodenbach (DE); Huthmacher, Klaus, Dr., 63584 Gelnhausen (DE); Shi, Nongyuan, Dr., 63512 Hainburg (DE); Markowz, Georg, Dr., 63791 Karlstein (DE); Häfner, Volker, 63508 Langenselbold (DE)

(56) Entgegenhaltungen:
- EP-A- 0 850 910
- EP-A- 0 916 642
- DE-A- 19 805 690

## Beschreibung

Die Erfindung betrifft ein Verfahren zur Herstellung von 2,3,5-Trimethylhydrochinondiestern durch Aromatisierung von 2,6,6-Trimethylcyclohex-2-en-1,4-dion (4-Oxo-Isophoron, Ketoisophoron, KIP) mit einem Acylierungsmittel und einem sauren löslichen oder unlöslichen Katalysator, das wahlweise kontinuierlich oder diskontinuierlich betrieben werden kann.

2,3,5-Trimethylhydrochinondiester sind wertvolle Zwischenprodukte bei der Synthese von Vitamin E und anderen Chromanverbindungen, die pharmazeutisch wirksam sind, aber auch in einem weiten Umfeld als Antioxydantien eingesetzt werden.

Die Bildung 2,3,5-Trimethylhydrochinondiestern aus KIP in Gegenwart eines stark sauren Katalysators und eines Acylierungsmittels wie Carbonsäureanhydride, Acylhalogenide oder Enolester ist bekannt und wird in mehreren Patentschriften (z. B. DE 26 46 172 C2, EP 808 815 A2 und EP 0 850 910 A1 ) beschrieben.

Es ist weiterhin bekannt, daß diese Aromatisierung zwar mit bis zu 100% Umsatz durchgeführt werden kann, die Selektivität jedoch, abhängig vor allem von dem Katalysator, zwischen 80 und 95 % liegt. Als Nebenprodukt wird hauptsächlich der isomere 3,4,5-Trimethylbrenzkatechindiester gebildet. Diese stets auftretende Nebenreaktion ist in EP 0 850 912 A1 beschrieben.

Die Isolierung und Reinigung der 2,3,5-Trimethylhydrochinondiester aus der Reaktionslösung ist verlustreich und aufwendig. In Beispielen mit gelösten Katalysatoren wird durch Zugabe von Wasser oder basischen, wäßrigen Lösungen das Gemisch aus 2,3,5-Trimethylhydrochinondiester und dem 3,4,5-Trimethylbrenzkatechindiester ausgefällt und durch Umkristallisation aus organischen Lösungsmitteln gereinigt. Die Rückgewinnung des Katalysators aus dem Filtrat ist aufwendig und bei der Umkristallisation wird die Ausbeute stark reduziert. Das bei der Aromatisierungsreaktion im Überschuß eingesetzte Acylierungsmittel hydrolisiert und ist für weitere Umsetzungen nicht verfügbar.

Bei Arbeiten mit ungelösten Katalysatoren, wie stark sauren Ionentauschern oder H-Y-Zeolithen, ist die Abtrennung und Wiederverwendung des Katalysators einfach, jedoch besteht bei der Isolierung und der verlustreichen Reinigung des 2,3,5-Trimethylhydrochinondiesters kein Unterschied zu den Reaktionslösungen mit gelösten Katalysatoren.

Diese Vorgehensweise ist aufgrund der geringen Isolierausbeute und der großen Abfallmenge für einen technischen Produktionsprozeß aus ökonomischer und ökologischer Sicht mit großen Nachteilen verbunden.

Aufgabe der Erfindung ist es, ein Verfahren bereitzustellen, mit dem 2,3,5-Trimethylhydrochinondiester wahlweise mit festen oder gelösten Katalysatoren bei minimaler Abfallmenge in einer kontinuierlichen oder diskontinuierlichen Fahrweise oder einer Kombination dieser Fahrweisen hergestellt werden kann.

Gegenstand der Erfindung ist ein Verfahren zur Herstellung von 2,3,5-Trimethylhydrochinondiester durch katalytische Umsetzung von Ketoisophoron (KIP) mit einem Acylierungsmittel, dadurch gekennzeichnet, daß man
a) als Acylierungsmittel ein Carbonsäureanhydrid mit einem C₁ bis C₄-Alkylrest einsetzt, nach der Umsetzung
b) gegebenenfalls einen Teil der entstandenen Carbonsäure abdestilliert,
c) die Reaktionslösung auf eine Temperatur von -10 bis 35°C, bevorzugt 0 bis 30°C, abkühlt,
d) das auskristallisierte Produkt abtrennt und wäscht und
e) das Filtrat in Schritt a) zurückführt, gegebenenfalls nach dem Abdestillieren eines Teils der entstandenen Carbonsäure und gegebenenfalls nach dem Ausschleusen eines Teils des Filtrats.

Bei der Umsetzung von KIP mit einem Carbonsäureanhydrid entstehen in Gegenwart eines stark sauren Katalysators 2,3,5-Trimethylhydrochinondiester, der isomere Trimethylbrenzkatechindiester und Carbonsäure. Pro mol umgesetztem KIP entstehen 2 mol Carbonsäure.

Es wurde gefunden, daß die bei der Reaktion entstehende Carbonsäure für die Trennung von 2,3,5-Trimethylhydrochinondiester und 3,4,5-Trimethylbrenzkatechindiester gut geeignet ist. Die beiden Aromaten können durch einfache Kristallisation aus Carbonsäuren, bevorzugt Essigsäure, getrennt werden. Es bilden sich trotz der Ähnlichkeit der Moleküle keine Mischkristalle.

Nach erfolgter Umsetzung wird die Reaktionslösung gekühlt und 2,3,5-Trimethylhydrochinondiester zur Kristallisation gebracht. Nach Abtrennen der Kristalle, wobei natürlich der gegebenenfalls verwendete feste Katalysator vorher abfiltriert wurde, verbleibt im Filtrat, entsprechend der nicht ausfiltrierte Anteil des 2,3,5-Trimethylhydrochinondiesters, der als Nebenprodukt gebildete 3,4,5-Trimethylbrenzkatechindiester, der gegenenfalls gelöst vorliegende Katalysator Katalysator sowie das im Überschuß eingesetzte Carbonsäureanhydrid. Dieses als Kreislauflösung bezeichnete Filtrat, eine gesättigte Produktlösung mit allen Nebenprodukten, nicht umgesetzten Edukten und gegebenenfalls dem Katalysator, wird vollständig recycliert, wobei bei homogener Reaktion eine erneute Zugabe des Katalysators entfällt. Das Acylierungsmittel kann jetzt in stöchiometrischer Menge eingesetzt werden, der für einen vollständigen Umsatz von KIP erforderliche Überschuß ist in dem recyclierten Filtrat enthalten.

Bei jeder Recyclierung vergrößert sich das Volumen der Lösung durch die Neubildung von 2 mol Carbonsäure pro mol umgesetztes KIP, wobei aufgrund des guten Lösevermögens nur ein Teil des gebildeten Diesters auskristallisiert. Deshalb wird vorzugsweise ein Teil der Carbonsäure abdestilliert und die Menge der Kreislauflösung so auf einem konstanten Volumen gehalten. Der Destillationsschritt kann wahlweise jeweils vor oder nach dem Abtrennen der Kristalle erfolgen.

Der Trimethylbrenzkatechindiester, in geringem Umfang auch andere Nebenprodukte, reichert sich in der Kreislauflösung an. Die Konzentration an Trimethylhydrochinondiester kann ohne Nachteil bis nahe der Sättigungskonzentration ansteigen. Dieser Wert ergibt sich aus der Löslichkeitskurve und der Kristallisationstemperatur. Im Falle des Diacetates kann bei einer Kristallisationstemperatur von 20°C die Konzentration an Trimethylbrenzkatechindiacetat in der Kreislauflösung bis auf 20 Gew.-% steigen, wobei die Reinheit des isolierten Produktes erhalten bleibt. Durch Ausschleusen eines Teilstromes der Kreislauflösung kann das Niveau an Nebenprodukten in der Lösung auf einer tolerablen Höhe gehalten werden. Die darin enthaltenen Katalysator- und Acylierungsmittelmengen werden durch entsprechende Zugabe in den Kreislauf ergänzt.

Bei dem erfindungsgemäßen Verfahren zur Herstellung von Trimethylhydrochinondiestern kommt ein weiterer Vorteil zum Tragen. Mit steigender Konzentration an Trimethylbrenzkatechindiacetat in der Kreislauflösung nimmt die Selektivität für die Neubildung dieses Nebenproduktes ab. Mit dem Ansteigen der Konzentration von 0 auf 15 G% in der Kreislauflösung wird die Selektivität für dessen Neubildung nahezu halbiert.

Das Waschen der Kristalle des gewünschten Produkts erfolgt mit der entsprechenden Carbonsäure, einer gesättigten Produktlösung in der Carbonsäure oder auch mit einem inerten Lösungsmittel, wie z.B. Kohlenwasserstoffen oder Ethern, das destillativ zurückgewonnen wird.

In dem erfindungsgemäßen Verfahren können alle Verfahrensschritte wahlweise so ausgelegt werden, daß der Betrieb kontinuierlich, batchweise oder in einer Kombination daraus erfolgt.

Das Verfahren besteht aus den Teilschritten: Chemische Umsetzung (I), Destillation (II), Kristallisation (III) und Feststoffabtrennung (IV). Dabei können die Teilschritte II und III zu einem gemeinsamen Schritt zusammengefaßt werden. Die Reihenfolge kann auch so gewählt werden, daß nach der chemischen Reaktion (I) zuerst die Kristallisation (III) und Feststoffabtrennung (IV) und danach die Destillation (II) erfolgt.

In den Reaktor für die chemische Umsetzung werden die Edukte, Ketoisophoron und ein Acylierungsmittel, vorzugsweise Essigsäureanhydrid, und die Kreislauflösung eingetragen. Die Art des Reaktors wird durch die Eigenschaften des eingesetzten Katalysators beeinflußt und kann beispielsweise ein Reaktor mit einem Festbett, ein einfacher Kessel oder ein Mischgefäß mit anschließender Verweilzeitstrecke sein. Die Menge an Kreislauflösung kann im Bereich von 4 bis 20 kg pro kg eingesetztes Ketoisophoron, vorzugsweise bei 5 bis 10 kg, liegen und soll so bemessen sein, daß der Kristallbrei nach der Kristallisation fließfähig ist.

Die benötigte Verweilzeit ist von der Art und der Menge des eingesetzten Katalysators und der gewählten Temperatur abhängig, wobei die Bedingungen vorzugsweise so gewählt werden, daß eine Verweilzeit von 1 bis 3 Stunden ausreicht. Da bei dem erfindungsgemäßen Verfahren die in dem Filtrat enthaltenen überschüssigen Edukte recycliert werden, kann der Umsatz des Ketoisophorons unter 100 Prozent liegen.

Die Destillation der Carbonsäure, bei bevorzugten Einsatz von Essigsäureanhydrid, der Essigsäure, erfolgt zweckmäßigerweise kontinuierlich oder diskontinuierlich über eine Kolonne, derer Trennleistung ausreicht, daß das Carbonssäureanhydrid im Sumpf bleibt. Dabei wird soviel Destillat abgenommen, daß das Volumen der Kreislauflösung konstant bleibt.

Beim Abkühlen der Reaktionslösung kristallisiert der Trimethylhydrochinondiester aus, der Trimethylbrenzkatechindieseter bleibt gelöst. Die Temperatur für den Kristallisationsvorgang kann zwischen -10 und +35°C liegen, wobei der Bereich von 0 bis 30°C bevorzugt ist. Die Kristallisation kann ein oder mehrstufig durchgeführt werden. Der Verfahrensschritt der Kristallisation erfolgt auf bekannte Weise kontinuierlich oder diskontinuierlich, wobei, durch die Neigung der Lösung zur Übersättigung, der Zusatz von Impfkristallen vorteilhaft ist.

Die Feststoffabtrennung erfolgt mit handelsüblichen Aggregaten kontinuierlich oder diskontinuierlich, wobei auf Feuchtigkeitsausschluß zu achten ist, um den im Filtrat enthaltenen Überschuß an Acylierungsmittel nicht zu hydrolysieren. Um die anhaftende Mutterlauge zu verdrängen ist ein Waschvorgang erforderlich. Als Waschflüssigkeit kann zum Beispiel ein Teil der abdestillierten Carbonsäure, ein inertes organisches Lösungsmittel wie Petroleumbenzin oder vorteilhaft eine Lösung von Trimethylhydrochinondiester in der Carbonsäure eingesetzt werden.

Das Filtrat wird zusammen mit den überschüssigen Edukten und gegebenenfalls mit dem gelösten Katalysator und den Nebenprodukten recycliert. Der Trimethylbrenzkatechindiester kann ohne Nachteile auf Werte bis zu 20 Gewichtsprozent im Filtrat ansteigen. Um ein nachhaltig sauberes Produkt zu erhalten, wird erfindungsgemäß nach mehrmaligem Recyclieren des Filtrates ein Teil des Filtrates ausgeschleust. Dabei wird die Menge ausgeschleust, in der die bei einem Cyclus neu gebildete Menge an dem Brenzkatechindiester gelöst ist.

Das folgende Beispiel soll das erfindungsgemäße Verfahren veranschaulichen, ohne einschränkend zu wirken.

### Beispiel 1

Das Reaktionsgefäß besteht aus einem thermostatisierten Doppelmantelgefäß mit Rührer, Überlauf und Eingängen für die Kreislauflösung, KIP und Essigsäureanhydrid. Pro Stunde werden 76 g KIP, 110 g Essigsäureanhydrid und 500 g Kreislauflösung eingepumpt. Die Kreislauflösung enthält 31% Trimethylhydrochinondiacetat, 17% Trimethylbrenzkatechindiacetat, 0,8% Trifluormethansulfonsäure, 38% Essigsäure und 10% Essigsäureanhydrid. Das Gefäß wird so thermostatisiert, daß die Innentemperatur 50 bis 55°C beträgt. Die homogene Lösung vom Überlauf wird durch ein auf 55°C thermostatisiertes Rohr als Nachreaktor geleitet. Das Gesamtvolumen von Reaktionsgefäß und Nachreaktor beträgt 1,4 1.

Die Reaktionslösung wird in eine Destillationsapparatur eingeleitet und bei 30 mbar werden stündlich 58 g Essigsäure abdestilliert.

Der Sumpf der Destillation wird der Kristallisation zugeführt und unter Rühren auf 20°C gekühlt. Nach jeweils 5 Stunden wird die erhaltene Suspension filtriert und die anhaftende Lösung durch Waschen mit 220 g einer gesättigten Lösung von Trimethylhydrochinondiacetat in Essigsäure verdrängt. Vom Filtrat werden 4 % ausgeschleust. Das verbleibende Filtrat wird zusammen mit 1,3 g frischem Katalysator erneut in das Reaktionsgefäß eindosiert.

Der Feststoff wird im Vakuum getrocknet. Das erhaltene Trimethylhydrochinondiacetat hat eine Reinheit von >98%, die Isolierausbeute beträgt 88%, bezogen auf eingesetztes Ketoisophoron.

## Patentansprüche

1. Verfahren zur Herstellung von 2,3,5-Trimethylhydrochinondiester durch katalytische Umsetzung von Ketoisophoron (KIP) mit einem Acylierungsmittel,
**dadurch gekennzeichnet,**
**daß** man
a) als Acylierungsmittel ein Carbonsäureanhydrid mit einem C₁ bis C₄-Alkylrest einsetzt, nach der Umsetzung
b) gegebenenfalls einen Teil der entstandenen Carbonsäure abdestilliert,
c) die Reaktionslösung auf eine Temperatur von -10 bis 35°C, bevorzugt 0 bis 30°C, abkühlt,
d) das auskristallisierte Produkt abtrennt und wäscht und
e) das Filtrat in Schritt a) zurückführt, gegebenenfalls nach dem Abdestillieren eines Teils der entstandenen Carbonsäure und gegebenenfalls nach dem Ausschleusen eines Teils des Filtrats.

2. Verfahren gemäß Anspruch 1,
**dadurch gekennzeichnet,**
**daß** man bei der Verwendung eines festen Katalysators diesen vor Schritt c) abtrennt.

3. Verfahren gemäß Anspruch 1 oder 2,
**dadurch gekennzeichnet,**
**daß** man als Acylierungsmittel Acetanhydrid oder Propionsäureanhydrid verwendet.

4. Verfahren gemäß einem oder mehreren der vorhergehenden Anspruche,
**dadurch gekennzeichnet,**
**daß** man die Reaktion im Kreislauf führt.

5. Verfahren gemäß Anspruch 1,
**dadurch gekennzeichnet,**
**daß** man einen Teil der Carbonsäure abdestilliert und das Volumen der Kreislauflösung auf einem konstanten Wert hält.

6. Verfahren gemäß Anspruch 1,
**dadurch gekennzeichnet,**
**daß** durch Ausschleusen eines Teils der Kreislauflösung die Konzentration des Trimethylbrenzkatechins in der Reaktionslösung bei der Kristallisationstemperatur unter der Löslichkeitsgrenze gehalten wird.

7. Verfahren gemäß Anspruch 4,
**dadurch gekennzeichnet,**
**daß** man bei 0 bis 30°C das Produkt auskristallisiert (Schritt c)).

8. Verfahren gemäß einem oder mehreren der vorhergehenden Ansprüchen,
**dadurch gekennzeichnet,**
**daß** man die Menge der Kreislauflösung so bemißt, daß der Kristallbrei nach der Kristallisation fließfähig ist.

9. Verfahren gemäß einem oder mehreren der vorhergehenden Ansprüchen,
**dadurch gekennzeichnet,**
**daß** man das Verfahren kontinuierlich, teilkontinuierlich oder batchweise durchführt.

## Claims

1. Process for the production of 2,3,5-trimethylhydroquinone diester by catalytic reaction of ketoisophorone (KIP) with an acylation agent,
**characterised in that**
a) a carboxylic acid anhydride with a C₁ to C₄ alkyl group is used as the acylation agent, after the reaction
b) optionally some of the carboxylic acid formed is distilled off,
c) the reaction solution is cooled to a temperature of -10 to 35°C, preferably 0 to 30°C,
d) the product that has crystallised out is separated off and washed and
e) the filtrate is fed back to step a), optionally after distilling off some of the carboxylic acid formed and optionally after sluicing out some of the filtrate.

2. Process according to claim 1,
**characterised in that** if a solid catalyst is used, it is separated off before step c).

3. Process according to claim 1 or 2,
**characterised in that** acetic anhydride or propionic acid anhydride is used as the acylation agent.

4. Process according to one or more of the preceding claims,
**characterised in that** the reaction is recirculated.

5. Process according to claim 1,
**characterised in that** some of the carboxylic acid is distilled off and the volume of the circulation solution is maintained at a constant value.

6. Process according to claim 1
**characterised in that**, by sluicing out some of the circulation solution, the concentration of trimethyl catechol in the reaction solution at the crystallisation temperature is kept below the solubility limit.

7. Process according to claim 4
**characterised in that** the product is crystallised out at 0 to 30°C (step C).

8. Process according to one or more of the preceding claims,
**characterised in that** the quantity of the circulation solution is measured in such a way, that the crystal pulp is flowable after crystallisation.

9. Process according to one or more of the preceding claims,
**characterised in that** the process is carried out continuously, partly continuously or batch-wise.

## Revendications

1. Un objet de l'invention réside dans un procédé pour la préparation de diesters de 2,3,5-triméthylhydroquinone par transformation catalytique de cétoisophorone (CIP) avec un agent d'acylation, **caractérisé**
a) en ce qu'on utilise comme agent d'acylation un anhydride de l'acide carboxylique présentant un radical alkyle en C₁ à C₄,
en ce qu'après la transformation
b) on élimine par distillation, le cas échéant, une partie de l'acide carboxylique formé,
c) on refroidit la solution réactionnelle à une température de -10 °C à 35 °C, de préférence de 0 °C à 30 °C,
d) on sépare et lave le produit cristallisé,
e) on recycle le filtrat dans l'étape a), le cas échéant après élimination par distillation d'une partie de l'acide carboxylique formé et le cas échéant après évacuation d'une partie du filtrat.

2. Procédé selon la revendication 1, **caractérisé en ce que**, dans le cas de l'utilisation d'un catalyseur solide, on sépare celui-ci avant l'étape c).

3. Procédé selon la revendication 1 ou 2, **caractérisé en ce qu'**on utilise de l'anhydride acétique ou de l'anhydride de l'acide propionique comme agent d'acylation.

4. Procédé selon l'une ou plusieurs des revendications précédentes, **caractérisé en ce qu'**on effectue la réaction dans un circuit.

5. Procédé selon la revendication 1, **caractérisé en ce qu'**on élimine une partie de l'acide carboxylique et qu'on maintient le volume de la solution en circulation à une valeur constante.

6. Procédé selon la revendication 1, **caractérisé en ce que** la concentration en triméthylpyrocatéchine dans la solution réactionnelle à la température de cristallisation est maintenue au-dessous de la limite de solubilité par évacuation d'une partie de la solution en circulation.

7. Procédé selon la revendication 4, **caractérisé en ce qu'**on cristallise le produit à 0 °C jusqu'à 30 °C (étape c).

8. Procédé selon l'une ou plusieurs des revendications précédentes, **caractérisé en ce qu'**on dimensionne la quantité de la solution en circulation de telle manière que le moût de cristallisation peut s'écouler après la cristallisation.

9. Procédé selon l'une ou plusieurs des revendications précédentes, **caractérisé en ce qu'**on réalise le procédé en continu, de manière partiellement continue ou par lot.
